(19) Europäisches Patentamt
European Patent Office
Office européen des brevets

(11) Publication number: 0 371 730
A1

# (12) EUROPEAN PATENT APPLICATION

(21) Application number: 89312294.5

(22) Date of filing: 27.11.89

(51) Int. Cl.5: C07D 233/84, A61K 31/415, C07D 233/64

Claims for the following Contracting States: ES + GR.

(30) Priority: 01.12.88 US 278622

(43) Date of publication of application: 06.06.90 Bulletin 90/23

(84) Designated Contracting States: AT BE CH DE ES FR GB GR IT LI LU NL SE

(71) Applicant: SMITHKLINE BEECHAM CORPORATION
P.O. Box 7929 1 Franklin Plaza
Philadelphia Pennsylvania 19101(US)

(72) Inventor: Kruse, Lawrence Ivan
9 Great Valley Parkway
Malvern Pennsylvania 19355(US)
Inventor: Ross, Stephen Torey
718 Old State Road
Berwyn Pennsylvania 19312(US)

(74) Representative: Waters, David Martin, Dr. et al
Smith Kline & French Laboratories Ltd.
Patent Department Mundells
Welwyn Garden City Hertfordshire AL7 1EY(GB)

## (54) Dopamine-B-Hydroxylase inhibitors.

(57) Dopamine-β-hydroxylase inhibitors of structure:

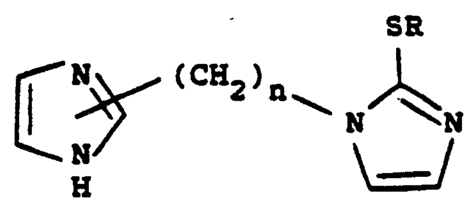

pharmaceutical compositions containing them and their use in methods of lowering blood pressure in mammals.

EP 0 371 730 A1

## DOPAMINE-β-HYDROXYLASE INHIBITORS

### FIELD OF THE INVENTION

This invention relates to novel compounds that inhibit dopamine-β-hydroxylase

### BACKGROUND OF THE INVENTION

In the catecholamine biosynthetic pathway, tyrosine is converted in three steps to norepinephrine (NE). Intermediates are dihydroxyphenylalanine (DOPA) and dopamine (DA). Dopamine is hydroxylated to norepinephrine by dopamine-β-hydroxylase (DBH) in the presence of oxygen and ascorbic acid.

Inhibition of catecholamine activity decreases blood pressure. Weinshilboum, Mayo Clin. Proc. 55, 39 (1980), reviews compounds that inhibit catecholamine activity by acting upon adrenergic receptors. Alternatively, the catecholamine biosynthetic pathway can be suppressed at any of the three steps, resulting in reduced NE levels. In addition to producing an antihypertensive effect, inhibitors of NE synthesis are active as diuretics, natriuretics, cardiotonics, and vasodilators. Inhibition of DBH activity can have the added advantage of increasing DA levels, which as reported by Ehrreich et al., "New Antihypertensive Drugs," Spectrum Publishing, 1976, pp.409-432, has selective vasodilator activity at certain concentrations.

DBH inhibitors also have been shown to reduce or prevent formation of gastric ulcers in rats by Hidaka et al., "Catecholamine and Stress," edit. by Usdin et al., Permagin Press, Oxford, 1976, pp.159-165 and by Osumi et al., Japan J. Pharmacol. 23, 904 (1973).

A number of DBH inhibitors are known. These generally are divided into two classes, namely, metal chelating agents, which bind copper in the enzyme, and phenethylalamine analogues. Rosenberg et al., "Essays in Neurochemistry and Neuropharmacology," Vol. 4, ed. by Youdim et al., John Wiley & Sons, 1980, pp.179-192, and Goldstein, Pharmacol. Ref. 18(1), 77 (1966), review DBH inhibitors.

Known DBH inhibitors include:

(a) 5-alkylpicolinic acids [See, Suda et al., Chem. Pharm. Bull. 17, 2377 (1969); Umezawa et al., Biochem. Pharmacol. 19, 35 (1969); Hidaka et al., Mol. Pharmacol. 9, 172 (1973); Miyano et al., Chem. Pharm. Bull. 26, 2328 (1978); Miyano et al., Heterocycles 14, 755 (1980); Claxton et al., Eur. J. Pharmacol. 37, 179 (1976)];

(b) BRL 8242 [see Claxton et al., Eur. J. Pharmacol. 37, 179 (1976)];

(c) 1-alkylimidazole-2-thiols [See, Hanlon et al., Life Sci. 12, 417 (1973); Fuller et al., Adv. Enzyme Requl. 15, 267 (1976)];

(d) substituted thioureas [See, Johnson et al., J. Pharmacol. Exp. Ther. 168, 229 (1969)]; and

(e) benzyloxyamine and benzylhydrazine [See, Creveling et al., Biochim. Biophys. Acta 64, 125 (1962); Creveling et al., Biochim. Bionhys. Acta 8, 215 (1962); Van Der Schoot et al., J. Pharmacol. Exp. Ther. 141, 74 (1963); Bloom, Ann. N.Y. Acad. Sci. 107, 878 (1963)];

(f) fusaric acid derivatives and analogues as reported by Runti et. al. in Il Farmaco Ed. Sci. 36, 260 (1980). These derivatives include phenylpicolinic acid, which has twice the inhibitory activity of fusaric acid, and 5-(4-chlorobutyl) picolinic acid, and others such as substituted amides of fusaric acid and acids and amides of 5-butyroylpicolinic acid, 5-aminopicolinic acid and 5-hydrazinopicolinic acid, and derivatives thereof;

(g) 5-(3,4-dibromobutyl)picolinic acid and 5-(dimethyldithiocarbamoylmethyl)picolinic acid (Hidaka et al., Molecular Pharmacology 9, 172-177, 1972);

(h) Bupicomide, 5-(n-butyl)picolinamine, Ehrreich et al., "New Antihypertensive Drugs", Spectrum Publications, 1976, pg. 409-432, reported as a DBH inhibitor that has antihypertensive activity;

(i) a series of 1-phenyl and 1-phenylalkylimidazole compounds having a mercapto or alkylthio group in the 2-position reported in European Patent Application No. 125,033 (published November 14, 1984);

(j) Methylpyridine derivatives isolated from the fermentation broth of a strain of Streptoverticillium (United States Patent No. 4,487,761); and

(k) 1-Benzyl-2-aminomethylimidazole derivatives (United States Patent No. 4,532,331).

However, non-specific, often toxic effects to known DBH inhibitors have obviated clinical use of these compounds. Fusaric acid, for example, is hepatotoxic. See, for example. Teresawa et al., Japan. Cir. J. 35, 339 (1971) and references cited therein.

## SUMMARY OF THE INVENTION

The present invention relates to 1-imidazolylalkylimidazole-2-thiols which have been found to be potent and prolonged inhibitors of DBH.

Presently preferred compounds of the invention include:
1-(2-imidazolylmethyl)-1,3-dihydro-2H-imidazole-2-thiol.

In addition the invention relates to intermediates useful in the preparation of the compounds, to pharmaceutical compositions comprising the compounds of the invention and a method of inhibiting DBH activity in mammals, including humans, which comprises administering internally to a subject an effective amount of a compound of the invention.

## DETAILED DESCRIPTION OF THE INVENTION

The present invention relates to compounds of structure (I) :

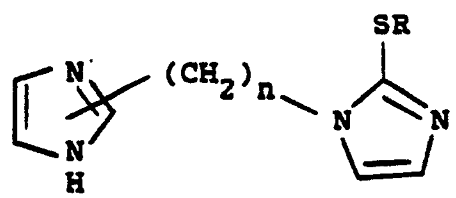

(I)

in which
R is hydrogen or $C_{1-4}$alkyl; and
n is 1 to 5;
and pharmaceutically acceptable salts and hydrates thereof.

Suitably, n is 1 to 5, preferably n is 1 or 2;

Suitably, R is hydrogen or $C_{1-4}$alkyl, preferably R is hydrogen.

Preferably, the $(CH_2)_n$ group is attached to the 2-position of the imidazole ring.

$C_{1-4}$alkyl means a straight or branched chain alkyl having from 1 to 4 carbons.

It will be apparent that compounds of structure (I) in which R is hydrogen can exist in a number of tautomeric forms i.e.

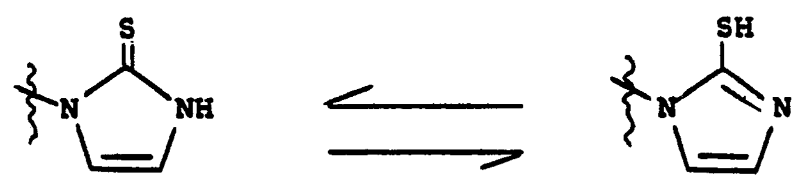

It is intended that structure (I) includes such tautomeric forms.

The compounds of structure (I) can be prepared by processes analogous to those known in the art, in particular compounds of structure (I) can be prepared by reacting a compound of structure (II):

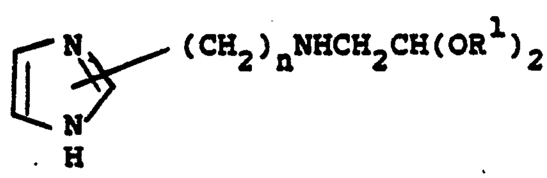

(II)

in which $R^1$ is $C_{1-4}$alkyl and n is 1 to 5 with an alkali metal thiocyanate and optionally thereafter alkylating

the compound of structure (I) so formed in which R is hydrogen to form a compound of structure (I) in which R is $C_{1-4}$alkyl and optionally forming a pharmceutically acceptable salt or hydrate thereof.

The reaction between the compound of structure (II) and the alkali metal thiocyanate is carried out in an inert solvent, preferably in the presence of an acid catalyst. Suitably, the thiocyanate is lithium or sodium thiocyanate, preferably potassium thiocyanate.

Suitably the acid catalyst is a mineral acid, preferably hydrochloric acid. Suitably the solvent is an inert solvent such as tetrahydrofuran or dioxane, or an ether or $C_{1-4}$alkanol. Preferably the solvent is ethanol. Suitably the reaction is conducted at a temperature of between ambient and reflux temperature of the solvent used for a period long enough to allow complete reaction.

The compounds of structure (II) which are novel and form a further aspect of the invention can be prepared from compounds of structure (III):

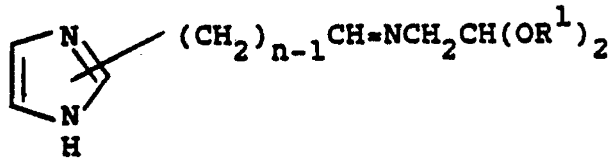

(III)

in which $R^1$ is $C_{1-4}$alkyl and n is 1 to 5 by reaction with a reducing agent in an inert solvent. Suitable reducing agents will be apparent to those skilled in the art and include, for example alkali metal hydrides such as lithum aluminium hydride or sodium borohydride or hydrogen in the presence of a noble metal catalyst such as palladium or platinum. Suitable solvents include $C_{1-4}$alkanols, in particular methanol.

The compounds of structure (III) which are also novel and form part of the invention can be prepared by reaction of a compound of structure (IV):

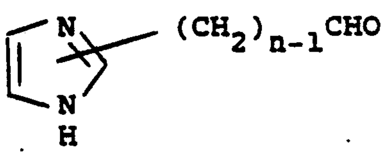

(IV)

$NH_2CH_2CH(OR^1)_2$ (V)

in which n is 1 to 5 with a suitable acetal of structure (V) in which $R^1$ is $C_{1-4}$alkyl, in a suitable solvent at elevated temperature. Suitable solvents include, for example, aromatic hydrocarbon solvents such as benzene, in particular xylene.

It will be appreciated that

- compounds of structure (I) in which R is $C_{1-4}$alkyl can be prepared by alkylating a compound of structure (I) in which R is hydrogen using an appropriate $C_{1-4}$alkylhalide such as methyliodide or butyl bromide with or without base such as $C_{1-4}$alkoxide or a tertiary amine such as triethylamine in a $C_{1-4}$alkanol such as methanol; and
- pharmaceutically acceptable acid addition salts of compounds of structure (I) can be formed with appropriate organic or inorganic acids by methods known in the art. For example, the free base can be reacted with a suitable inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Exemplary of such salts are the maleate, fumarate, lactate, oxalate, methanesulfonate, ethanesulfonate, benzenesulfonate, tartrate, citrate, hydrochloride, hydrobromide, sulfate, phosphate, quinate and nitrate salts.

The compounds of structure (I) and pharmaceutically acceptable salts or hydrates thereof have been found to be inhibitors of DBH activity, and as such they are useful as diuretic, natriuretic, cardiotonic, antihypertensive, and vasodilator agents, as well as anti-ulcerogenic and anti- Parkinsonian agents. In a further aspect the present invention therefore provides a method of inhibiting DBH activity in mammals which comprises administering to a subject in need thereof an effective amount of a compound of structure (I).

Listed in Table I are compounds of the invention that were tested for in vitro DBH inhibition by a standard procedure for assaying conversion of tyramine to octopamine in the presence of DBH. J.J. Pisano, et al., Biochim. Biophys. Acta, 43, 566-568 (1960). Octopamine was assayed following sodium periodate

oxidation to p-hydroxybenzaldehyde by measuring spectrophotometric absorbance at 330 nm. In Table I, inhibition is given in molar concentration of compound at which DBH activity was halved ($IC_{50}$). Fusaric acid, by this test has an $IC_{50}$ of $8 \times 10^{-7}$M.

Table I

| Compound | DBH $IC_{50}$ (M) |
|---|---|
| 1-(2-imidazolylmethyl)-1,3-dihydro-2H-imidazole-2-thione | $3.3 \times 10^{-5}$ |

Further, spontaneously hypertensive rats were treated with 1-(2-imidazolylmethyl)-1,3-dihydro-2H-imidazole-2-thione at a dose of 50 mg/kg intraperitoneally, and mean arterial blood pressure was monitored for 250 minutes using indwelling cannulae in the tail arteries. When compared to vehicle-treated controls, the animals treated with this compound exhibited significant blood pressure reductions within 30 minutes following treatment and exhibited their lowest blood pressures when monitoring was discontinued. The maximal blood pressure reduction was approximately 40 mmHg.

When used in the method of the present invention the compounds are incorporated into standard pharmaceutical compositions. In a further aspect the present invention provides pharmaceutical compositions comprising a compound of structure (I) or a pharmaceutically acceptable salt or hydrate thereof and a pharmaceutically acceptable carrier.

The compounds of structure (I) can be incorporated into convenient pharmaceutical dosage forms such as capsules, tablets, or injectable preparations. Solid or liquid pharmaceutical carriers can be employed. Solid carriers include, starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, along or with a wax. The amount of solid carrier varies widely but, preferably, will be from about 25 mg to about 1 g per dosage unit. When a liquid carrier is used, the preparation will be in the form of a syrup, elixir, emulsion, soft gelatin capsule, sterile injectable liquid, or an aqueous or nonaqueous liquid suspension.

The pharmaceutical preparations are made following conventional techniques of a pharmaceutical chemist involving mixing, granulating and compressing, when necessary, for tablet forms, or mixing, filling, and dissolving the ingredients, as appropriate to give the desired oral or parenteral products.

Doses of the present compounds of Structure (I) in a pharmaceutical dosage unit as described above will be an efficacious, non-toxic quantity selected from the range of 0.1-100 mg/kg of active compound, preferably 0.1-50 mg/kg.

The selected dose is administered to a human patient in need of DBH inhibition from 1-6 times daily, orally, rectally, by injection, or continuously by infusion. Oral dosage units for human administration preferably contain from 1 to 500 mg of active compound. Parenteral administration, which uses lower dosages is preferred. Oral administration, as higher dosages, however, also can be used when safe and convenient for the patient.

The following examples serve to illustrate the invention.

## Example 1

### 1-(2-Imidazolylmethyl)-1,3-dihydro-2H-imidazole-2-thione

A 2.5 g (0.026 mol) quantity of imidazole-2-carboxaldehyde and 2.73 g (0.026 mol) of aminoacetaldehyde dimethylacetal were stirred in 25 ml of toluene and the mixture heated to reflux using a Dean-Stark trap. Reflux was continued for one hour and then the mixture was cooled and the crystalline material which had precipitated was filtered to give 1.6 g (34% yield of imine). A 1.54 g (8.4 mmol) quantity of the imine was stirred in 15 ml MeOH and 0.32 g (8.4 mmol) of sodium borohydride was added in portions and the mixture was heated to reflux for one hour following the addition. The reaction mixture was cooled, diluted

with water and then was extracted three times with ethyl acetate. Concentration of the combined ethyl acetate extracts gave 0.97 g (5.2 mmol, 62%) of N-(2-imidazolylmethyl)aminoacetaldehyde dimethyl acetal. This was stirred with 0.51 g (5.2 mmol) potassium thiocyanate in a mixture of 5 ml ethanol, 10 ml water and 1 ml 12N hydrochloric acid and the mixture was heated to reflux for one hour and then was cooled. The aqueous mixture was neutralised to pH seven with sodium bicarbonate and then was extracted three times with ethyl acetate. The combined ethyl acetate extracts were concentrated to give 0.41 g (44%) of 1-2-imidazolylmethyl-1,3-dihydro-2H- imidazole-2-thione. This was dissolved in a little methanol and chromatrographed on a column of 40 g of flash silica gel using 10% methanol in chloroform to give 0.26 g of purified material, m.p. 214-216 °C dec. infrared, $^1$H-NMR and mass spectra and elemental analyses (C,H,N) were consistent with structure.

## Example 2

1-(4-Imidazolylmethyl)-1,3-dihydro-2H-imidazole-2-thione

The reaction of imidazole-4-carboxaldehyde as in Example 1 yields the title compound.

## Example 3

1-(2-Imidazolylethyl)-1,3-dihydro-2H-imidazole-2-thione

The reaction of imidazole-2-acetaldehyde as in Example 1 yields the title compound.

## Example 4

1-(2-Imidazolylpropyl)-1,3-dihydro-2H-imidazole-2-thione

The reaction of imidazole-2-propanol as in Example 1 yields the title compound.

## Example 5

1-(2-Imidazolylmethyl)-1,3-dihydro-2-methylthioimidazole

The reaction of 1-(2-imidazolylmethyl)-1,3-dihydro-2H-imidazole-2-thione with one equivalent of sodium methoxide and one equivalent of iodomethane in methanol at 0 °C yields the title compound.

## Example 6

An oral dosage form for administering the presently invention compounds is produced by screening, mixing, and filling into hard gelatin capsules the ingredients in the proportions shown in Table II, below:

Table II

| Compound | Amounts |
|---|---|
| 1-(2-Imidazolylmethyl)-1,3,-dihydro-2H-imidazole-2-thione | 50 mg |
| magnesium stearate | 5 mg |
| lactose | 75 mg |

## Example 7

The sucrose, calcium sulfate dihydrate, and compound shown in Table III below, are mixed and granulated in the proportions shown with a 10° % gelatin solution. The wet granules are screened, dried, mixed with the starch, talc and stearic acid, screened and compressed into a tablet.

Table III

| Compound | Amounts |
|---|---|
| 1-(2-Imidazolylmethyl)-1,3,-dihydro-2H-imidazole-2-thione | 100 mg |
| calcium sulfate dihydrate | 150 mg |
| sucrose | 20 mg |
| starch | 10 mg |
| talc | 5 mg |
| stearic acid | 3 mg |

## Example 8

The compound of Example 1 is dispersed in 25 ml of normal saline to prepare an injectable preparation.

**Claims**

1. A compound of the formula (I):

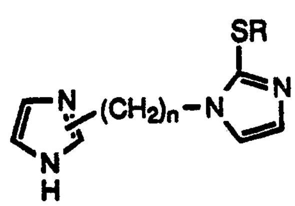

(I)

in which
R is hydrogen or $C_{1-4}$alkyl; and
n is 1 to 5;
or a pharmaceutically acceptable salt or hydrate thereof.

2. A compound according to claim 1 in which R is hydrogen.

3. A compound according to claim 1 or 2 in which n is 1 or 2.

4. A compound according to claim 1 which is 1-(2-imidazolylmethyl)-1,3-dihydro-2H-imidazole-2-thione.

5. A compound according to any one of claims 1-4 for use as a medicament.

6. A pharmaceutical composition comprising a compound according to any one of claims 1-4 in association with a pharmaceutically acceptable carrier.

7. A process for the preparation of a compound of formula (I) or a pharmaceutically acceptable salt or hydrate thereof as defined in claim 1, which comprises reacting a compound of formula (II): in which $R^1$ is $C_{1-4}$alkyl and n is 1 to 5 with an alkali metal thiocyanate and optionally thereafter alkylating the compound of formula (I) so formed in which R is hydrogen to form a compound in which R is $C_{1-4}$alkyl and optionally forming a pharmaceutically acceptable salt or hydrate thereof.

8. The use of a compound of formula (I) or a salt or hydrate thereof as defined in claim 1 in the manufacture of a medicament for inhibiting dopamine-$\beta$-hydroxylase.

9. A compound of formula (II):

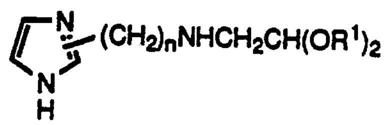

(II)

in which $R^1$ is $C_{1-4}$alkyl and n is 1 to 5.

10. A compound of formula (III):

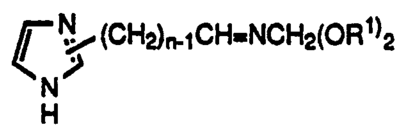

(III)

in which $R^1$ is $C_{1-4}$alkyl and n is 1 to 5.

Claims for the following Contracting State: GR

1. A process for the preparation of a compound of formula (I):

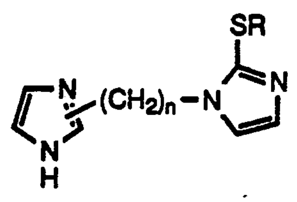

(I)

in which
R is hydrogen or $C_{1-4}$alkyl; and
n is 1 to 5;
or a pharmaceutically acceptable salt or hydrate thereof, which comprises reacting a compound for formula (II):

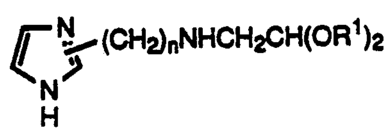

(II)

in which $R^1$ is $C_{1-4}$alkyl and n is 1 to 5 with an alkali metal thiocyanate and optionally thereafter alkylating the compound of formula (I) so formed in which R is hydrogen to form a compound of formula (I) in which R is $C_{1-4}$alkyl and optionally forming a pharmaceutically acceptable salt or hydrate thereof.

2. A process according to claim 1 for preparing a compound in which R is hydrogen.

3. A process according to claim 1 or 2 for preparing a compound in which n is 1 or 2.

4. A process according to claim 1 for preparing a compound which is 1-(2-imidazolylmethyl)-1,3-dihydro-2H-imidazole-2-thione.

5. A process for preparing 1-(2-imidazolylmethyl)-1,3-dihydro-2H-imidazole-2-thione or a pharmaceutically acceptable salt or hydrate thereof which comprises reacting N-(2-imidazolylmethyl)aminoacetaldehyde dimethyl acetal with potassium thiocyanate.

6. A process for preparing a pharmaceutical composition which comprises bringing into association a compound according to any one of claims 1 to 4 or a pharmaceutically acceptable salt or hydrate thereof and a pharmaceutically acceptable carrier.

7. The use of a compound of formula (I) or a pharmaceutically acceptable salt or hydrate thereof as defined in claim 1 in the manufacture of a medicament for inhibiting dopamine-$\beta$-hydroxylase.

8. A compound of formula (II):

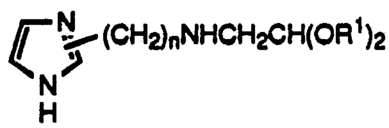

(II)

in which $R^1$ is $C_{1-4}$alkyl and n is 1 to 5.

9. A process for preparing a compound of the formula (II):

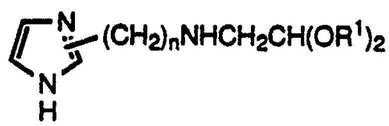

(II)

in which $R^1$ is $C_{1-4}$alkyl and n is 1 to 5, which comprises reacting a compound formula (III):

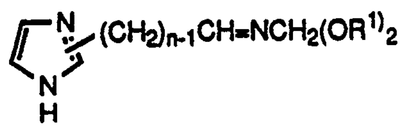

(III)

in which $R^1$ is $C_{1-4}$alkyl and n is 1 to 5 with an imine reducing agent.

10. A compound of formula (III):

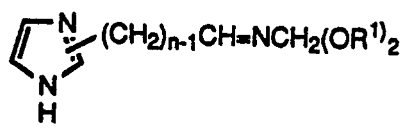

(III)

in which $R^1$ is $C_{1-4}$alkyl and n is 1 to 5.

Claims for the following Contracting State: ES

1. A process for the preparation of a compound of formula (I):

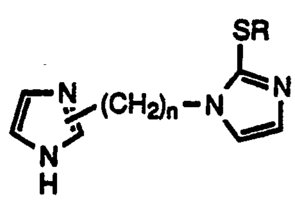

(I)

in which

R is hydrogen or $C_{1-4}$alkyl; and
n is 1 to 5;
or a pharmaceutically acceptable salt or hydrate thereof, which comprises reacting a compound for formula (II):

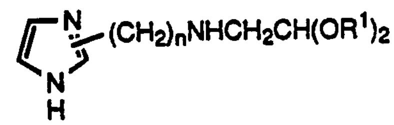

(II)

in which $R^1$ is $C_{1-4}$alkyl and n is 1 to 5 with an alkali metal thiocyanate and optionally thereafter alkylating the compound of formula (I) so formed in which R is hydrogen to form a compound of formula (I) in which R is $C_{1-4}$alkyl and optionally forming a pharmaceutically acceptable salt or hydrate thereof.

2. A process according to claim 1 for preparing a compound in which R is hydrogen.

3. A process according to claim 1 or 2 for preparing a compound in which n is 1 or 2.

4. A process according to claim 1 for preparing a compound which is 1-(2-imidazolylmethyl)-1,3-dihydro-2H-imidazole-2-thione.

5. A process for preparing 1-(2-imidazolylmethyl)-1,3-dihydro-2H-imidazole-2-thione or a pharmaceutically acceptable salt or hydrate thereof which comprises reacting N-(2-imidazolylmethyl)aminoacetaldehyde dimethyl acetal with potassium thiocyanate.

6. A process for preparing a pharmaceutical composition which comprises bringing into association a compound according to any one of claims 1 to 4 or a pharmaceutically acceptable salt or hydrate thereof and a pharmaceutically acceptable carrier.

7. The use of a compound of formula (I) or a pharmaceutically acceptable salt or hydrate thereof as defined in claim 1 in the manufacture of a medicament for inhibiting dopamine-$\beta$-hydroxylase.

8. A process for preparing a compound of the formula (II):

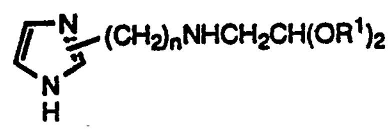

(II)

in which $R^1$ is $C_{1-4}$alkyl and n is 1 to 5, which comprises reacting a compound formula (III):

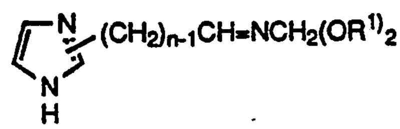

(III)

in which $R^1$ is $C_{1-4}$alkyl and n is 1 to 5 with an imine reducing agent.

9. A process for preparing a compound of the formula (III) :

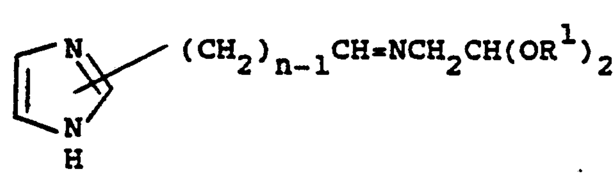

(III)

in which $R^1$ is $C_{1-4}$alkyl and n is 1 to 5, which comprises reacting a compound of the formula (IV) with an acetal of structure (V) :

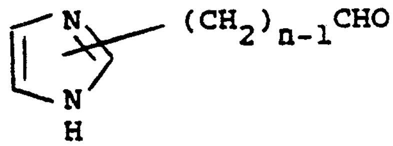

(IV)

$NH_2CH_2CH(OR^1)_2$ (V)

in which n and $R^1$ are as hereinbefore defined.

European Patent Office

# EUROPEAN SEARCH REPORT

Application Number

EP 89 31 2294

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X<br><br><br>X | EP-A-0 244 803 (MERRELL DOW PHARMACEUTICALS INC.)<br>* Pages 1-3; pages 6-7; form. VI *<br>* Page 12, line 24 - page 13, line 8 *<br>--- | 1-6<br><br><br>7-9 | C 07 D 233/84<br>A 61 K 31/415<br>C 07 D 233/64 |
| D,A | EP-A-0 125 033 (SMITHKLINE BECKMAN CORP.)<br>----- | | |
| | | | **TECHNICAL FIELDS SEARCHED (Int. Cl.5)** |
| | | | C 07 D 233/00<br>A 61 K 31/00 |

The present search report has been drawn up for all claims

| Place of search | Date of completion of the search | Examiner |
|---|---|---|
| THE HAGUE | 23-02-1990 | DE BUYSER I.A.F. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)